# EUROPEAN PATENT SPECIFICATION

(11) **EP 3 547 941 B1**
(45) Date of publication and mention of the grant of the patent: **11.01.2023**
(21) Application number: 17877192.9
(22) Date of filing: 12.11.2017
(51) Int. Cl.: A61B 17/34, A61M 5/178, A61B 1/05

(54) **A MEDICAL DEVICE HAVING A GAS PATH APPARATUS**
MEDIZINISCHE VORRICHTUNG MIT EINER GASWEGVORRICHTUNG
DISPOSITIF MÉDICAL COMPRENANT UN APPAREIL À TRAJET DE GAZ

(30) Priority: 30.11.2016 US 201662427825 P
(43) Date of publication of application: 09.10.2019
(73) Proprietor: 270 Surgical Ltd., 4250212 Netanya (IL)
(72) Inventor: LEVY, Avraham, 4691000 Kfar Shmaryahu (IL)
(74) Representative: Schulz Junghans Patentanwälte PartGmbB
(86) International application number: PCT/IL2017/051226
(87) International publication number: WO 2018/100571

(56) References cited:
- WO-A1-2012/012379
- WO-A2-2012/078872
- CN-U- 204 049 832
- US-A1- 2007 088 275
- US-A1- 2007 088 275
- US-A1- 2009 137 943
- US-A1- 2011 028 891
- US-A1- 2013 331 730
- US-A1- 2013 331 730
- US-A1- 2014 213 850

## Description

### FIELD OF THE INVENTION

The present invention generally relates to the field of medical instruments inserted into the patient's body and more specifically to the field of medical instruments designed to be inserted into the patient's body during medical procedures which require a stable insufflation level.

### BACKGROUND OF THE INVENTION

Insufflation is the act of filling something (such as a gas, powder, or vapor) into a body cavity. Insufflation has many medical uses, most notably as a route of administration for various drugs. Gases are often insufflated into a body cavity to inflate the cavity for more workroom, e.g. during laparoscopic surgery. A common gas used in this manner is carbon dioxide, because it is nonflammable, colorless and dissolves readily in blood. Smoke evacuation using insufflation is also a known method during medical procedures, to allow for clear image.

Insufflation systems typically contain a Gas supply and control system called an insufflator, sensors, gauges (usually embedded in the insufflator), a tube set and a body entry apparatus that is called "Gas path apparatus".

Medical procedures which require insufflation may differ from one another by the Gas path apparatus type and size utilized in such medical procedures. In some other cases, the Gas path apparatus may comprise gas entrance or exit ports located in different places at the Gas path apparatus surface. Furthermore, during certain medical procedures the Gas path apparatus type may need to change in order to meet different requirements in different stages of said medical procures. Insufflators are sometimes embedded in a complex, large and expensive system which includes balloons for storing the gas, valves, sensors and the like.

WO 2012/078872 A2 discloses a portable laparoscope. The portable laparoscope includes a housing and an elongated tube coupled to the housing. A lighting source and a camera are disposed proximate to an end of the elongated tube opposite the housing. The camera is configured to capture an image in a viewing area that is illuminated by light provided by the lighting source. The portable laparoscope includes an image display apparatus configured to display the images acquired by the camera and/or to transmit the images to a remote display device. The housing may be configured to hold and position an insufflator.

US 2013/0331730 A1 discloses an anatomical probe system comprises an elongated flexible body and an operational component extending within a channel of the flexible body. The system also comprises a support member at a distal end of the elongated flexible body. The support member includes a fluid director adapted to direct a fluid from the channel toward the operational component. The fluid may be automatically directed towards the operational component in response to detecting a material obstruction of an image.

US 2014/0213850 A1 discloses endoscopes, such as colonoscopes, that provide a broader field of view and allow extended access of surgical tools and also enable efficient packing of all necessary elements in the tip section, while maintaining their functionality. Also described are methods and systems for capturing and displaying still and video images using an endoscope corresponding to a left-side looking, a front-looking and a right-side looking viewing element of an endoscopic tip generated in a native aspect ratio.

US 2007/0088275 A1 discloses a trocar for use in a minimally invasive surgical procedure includes an elongated body, nozzle means and means for delivering a pressurized flow of fluid to the nozzle means. The elongated body has a generally tubular configuration with coaxially arranged inner and outer walls and longitudinally opposed proximal and distal end portions, with the inner wall defining a lumen to accommodate passage of an instrument therethrough. The nozzle means is operatively associated with the inner wall of the body for directing pressurized fluid into the lumen to develop a pressure differential in an area within a region extending from a location adjacent a distal end portion of the lumen to a location adjacent a proximal end portion of the lumen, to form a fluid seal around an instrument passing therethrough.

US 2011/0028891 A1 discloses a laparoscopic port device includes a compliant port body having a distal and proximal end having a lumen extending therethrough. The lumen has a filtering agent configured to retain or treat particulate contaminates present in insufflation gases. The laparoscopic port device further includes a valve operatively connected with the lumen to selectively regulate flow of the insufflation gases therethrough.

WO 2012/012379 A1 discloses air management control systems and methods maintain and manage an intra-abdominal gas environment during laparoscopic surgery. The systems and methods locate a plurality of in vivo sensors to monitor different environmental conditions within the operative space insufflated with pressurized C02, e.g., C02 insufflation airflow velocity, C02 pressure, aspiration airflow velocity, and at least one of humidity level, temperature, density of smoke/particulates, odors, and sound within the operative space. The systems and methods couple the plurality of in vivo sensors to a master controller. The master controller implements pre¬ programmed rules to generate control commands that govern the delivery of pressurized C02 and aspiration pressure into and out of the operative space in response, at least in part, to the different environmental conditions monitored by the in vivo sensors.

### SUMMARY OF THE INVENTION

The subject matter discloses a medical surgery imaging device designed to be inserted into a patient's body, comprising a tube, a surgical gas path apparatus located inside the tube and designed to maintain a gas pressure level within said cavity or hollow in a body, at least one camera located within said tube.

In some cases, the medical surgery imaging device maintains a gas pressure level by circulating gas between said body cavity and said surgical Gas path apparatus.

In some cases, the surgical gas path apparatus is attached to said tube.

In some cases, the surgical gas path apparatus comprises a gas exit port configured to release pressurized gas from the device. In some cases, the surgical gas path apparatus comprises a gas entry port which delivers pressurized gas into the body cavity. In some cases, the surgical gas path releases the gas received from said body cavity and release the gas from the surgery imaging device. In some cases, the surgical Gas path apparatus releases away the pressurized from received from said body cavity.

In some cases, the medical surgery imaging device further comprises a light source designed to illuminate the vicinity of the medical surgery imaging device.

The subject matter also discloses a medical surgery system controlling gas pressure within a body cavity, the system comprising a gas inlet connected to a gas source, a control system connected to the gas inlet and to a gas path apparatus supplying gas into a patient's body, a feedback module configured to receive an indication generated by a sensor of the medical imaging device located in the body cavity, wherein the control system is configured to control the gas flow from the gas inlet into the body cavity according to the indication.

In some cases, the sensor is a pressure sensor configured to detect pressure external to the medical imaging device and internal to the body cavity. In some cases, the sensor is a flow sensor configured to detect gas flowing in the surgical gas path apparatus. In some cases, the sensor is an image sensor.

In some cases, the indication from the image sensor identifies smoke in the image field of view and the control system automatically evacuates gas from the body cavity. In some cases, the indication from the image sensor identifies fog in the image field of view and the control system automatically transfers warm fluid into the body cavity. In some cases, the medical surgery system further comprises a signal processing system configured to receive the signal from the image sensor and generate the indication used by the control system.

The subject matter also discloses a two-piece medical surgery imaging device designed to be inserted into a patient's body, comprising, a tube, a surgical gas path apparatus attached to an external wall of the tube and designed to maintain a gas pressure level within said cavity or hollow in a body, at least one camera located within said tube, wherein the surgical gas path apparatus is designed to be replaced with another surgical gas path apparatus.

In some cases, the medical surgery imaging device maintains a gas pressure level by circulating gas between said body cavity and said surgical Gas path apparatus. In some cases, the surgical gas path apparatus is a reusable device. In some cases, the surgical gas path apparatus comprises a mechanism for attaching the surgical gas path apparatus to the device. The invention is defined in independent claim 1 with further embodiments being defined in dependent claims 2 - 8.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figs. 1A-1D disclose various configurations of medical systems having Medical Surgery Imaging Device (MSID) with an Gas path apparatus inserted in a body cavity and a system for maintaining the gas pressure within a body cavity or a body hollow, according to exemplary embodiments of the present invention;
Fig. 2 shows an MSID with surgical Gas path apparatus, according to exemplary embodiments of the present invention;
Fig. 3 shows a cross-section of an MSID with a Gas path apparatus and a gas delivery mechanism located at the lateral section of the MSID, not falling under the scope of the claimed subject-matter;
Fig. 4 shows a cross-section of an MSID with a Gas path apparatus and a gas delivery mechanism located at the front section of the MSID, not falling under the scope of the claimed subject-matter;
Fig. 5 shows a replaceable surgical Gas path apparatus in half cylinder-shaped tube designed to be attached to half-cylinder-shaped MSID and become a full cylinder-shaped MSID, according to exemplary embodiments of the present invention;
Figs. 6A and 6B show a surgical Gas path apparatus designed to be attached to a MSID, according to exemplary embodiments of the present invention;
Fig. 7 shows a lateral view of a MSID with a Gas path apparatus, according to exemplary embodiments of the present invention;
Fig. 8 demonstrates an angled view of a MSID with an Gas path apparatus, according to exemplary embodiments of the present invention; and,
Fig. 9 shows an angled view of MSID with a Gas path apparatus, according to exemplary embodiments of the present invention.

### DETAILED DESCRIPTION OF THE INVENTION

The present invention discloses a Medical Surgery Imaging Device (MSID) with a surgical Gas path apparatus. The MSID is designed to be inserted into a patient's body in order to maintain a stable insufflation level during certain medical procedures. Such medical procedures can include diverse types of operational acts which require insufflation maintenance and control, or can be driven by pneumoperitoneum. The MSID which with the surgical Gas path apparatus may comprise one or more cameras for capturing visual content such as images or video of a body tissue located in the vicinity of the MSID. The images captured by the MSID may be transmitted to a remote location, for example using a wireless transmitter or wired communication. The remote location may be a computer or a server, where the visual content may be displayed, analyzed, manipulated or otherwise processed. The MSID can also comprise illumination devices for example LEDs, to illuminate the area captured by the one or more cameras. The MSID may use an internal light source in case the illumination is implemented by transfer of light via a cable inside the MSID. The MSID can also comprise an internal power source, such as a battery configured to provide power to various components of the MSID. In some cases, the MSID may comprise an external power source which delivers power to the internal components of the MSID.

The MSID may be designed to be utilized for diverse medical procedures. In some cases, such medical procedures may be examining a body tissue, performing an endoscopy, biopsy, surgery, inject solutions, arthroscopy, and the like. In some other cases such medical procedures may be applications such as cardiology diagnostic tests, cardiovascular, neurological, gastrointestinal, neurovascular, ophthalmology procedures, and the like. In possible embodiments of the present invention, the medical procedures may include instruments used to examine the interior of a hollow organ or cavity of the body such as endoscope, laparoscope, rectoscope, a catheter and the like. In some other embodiments of the present invention, the MSID can be integrated into a robotic surgery used in a robot-assisted surgery.

The surgical Gas path apparatus which can be mounted or attached to the MSID may comprise a pressurized gas delivery mechanism in order to maintain the insufflation level in a body cavity. In some cases, the surgical Gas path apparatus may be utilized to support diverse medical procedures performed using the MSID, as disclosed above. The gas may be air, CO₂ or any other gas or fluid desired by a person skilled in the art.

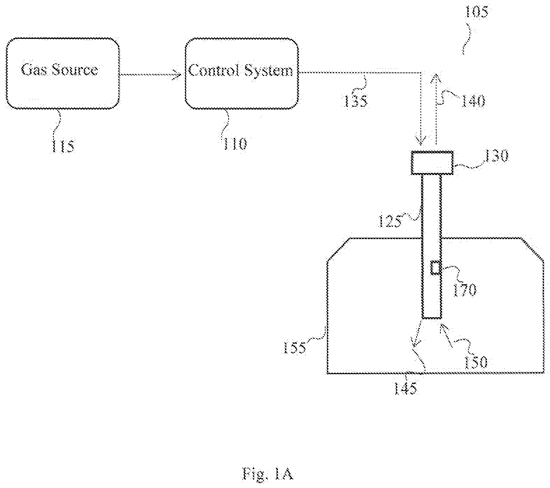

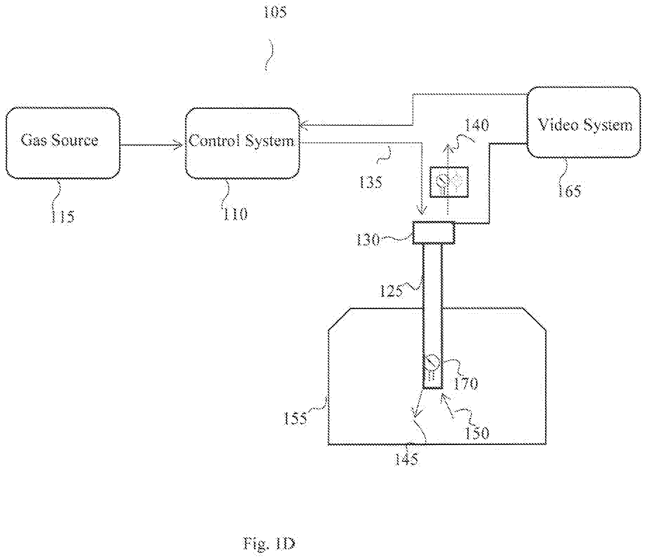

Fig. 1A discloses an MSID inserted in a body cavity and a system for maintaining and controlling the gas pressure and flow within the body cavity, according to exemplary embodiments of the present invention. The System 105 comprising an MSID 125 inserted into a body cavity 155. Such body cavity 155 may be a hollow or a native opening in the body which may need to be inflated by gas during a medical procedure. A distal end of the MSID 125 is used to output the gas into the body cavity 155 via output mechanism 145 and suck or release gas from the body cavity 155 using suction or relief mechanism 150, as detailed below.

System 105 also comprises a gas interface 130 designed to contain the gas entrance to the MSID 125, as shown in arrow 135 and the gas releasing as shown in arrow 140. System 105 also comprises a control system 110 configured to manage the pressure level of the gas within the body cavity. The control system 110 is configured to receive gas from gas source 115 and deliver it further to the gas interface 130. The system disclosed in figure 1A is passive and receives commands from an external source, for example a remote computer that sends commands to a receiver residing in the system 105.

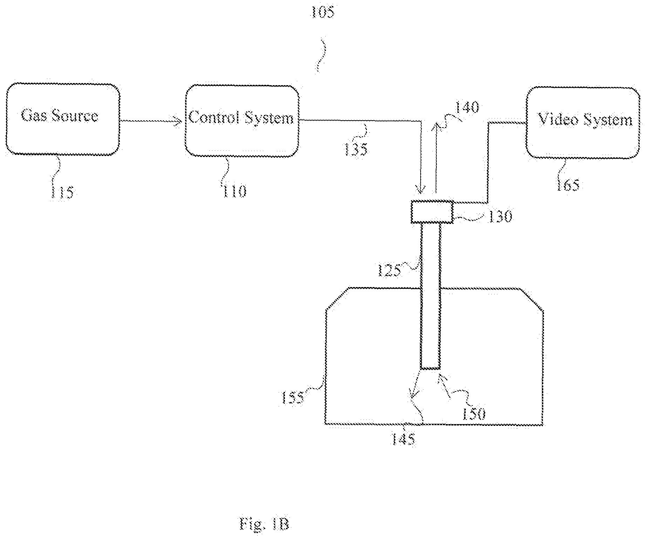

Fig. 1B discloses an MSID and a system for maintaining and controlling the gas pressure and flow within the body cavity, according to exemplary embodiments of the present invention. The MSID 125 communicates with the video system 165 that captures images or video of the vicinity of the MSID 125, for example in the body cavity filled with gas by the system 105. The images or video provided by the video system 165 may be analyzed by a processor of the MSID 125, to provide an indication concerning the area captured by the video system 165. The processor may adjust the gas flow in response to the images received from the video system 165, for example increase or decrease gas flow into the body cavity, remove gas, change the gas type, generate an alert to be sent to a medic's remote device, and the like.

In some cases, the MSID can determine that the insufflation level is too low by identifying the abdominal wall location. In such case, the MSID may the control system 110 to increase gas flow.

The MSID 125 also comprises an imaging unit configured to capture an image or video from the vicinity of the MSID 125 while maintaining and controlling the gas pressure and flow in the patient's tissue. The imaging unit may send the captured data to a remote unit via a wire located in the vicinity of MSID 125, along the tube used to transfer the gas. The MSID 125 may also comprises an illumination unit for illuminating the field of view captured by the imaging unit. The MSID 125 may also comprise medical instruments used to examine the interior of a hollow organ or cavity of the body and preform some medical procedures such as: biopsy, surgical act, tissue examination, imaging procedures, and the like.

The pressure and flow control of the system 105 may be adjusted to different medical needs. Such needs may include maintaining gas pressure, consistently circulating the gas or to discreetly circulate the gas as required. The system 105 may include mechanical, electrical or computerized modules to perform the tasks disclosed above.

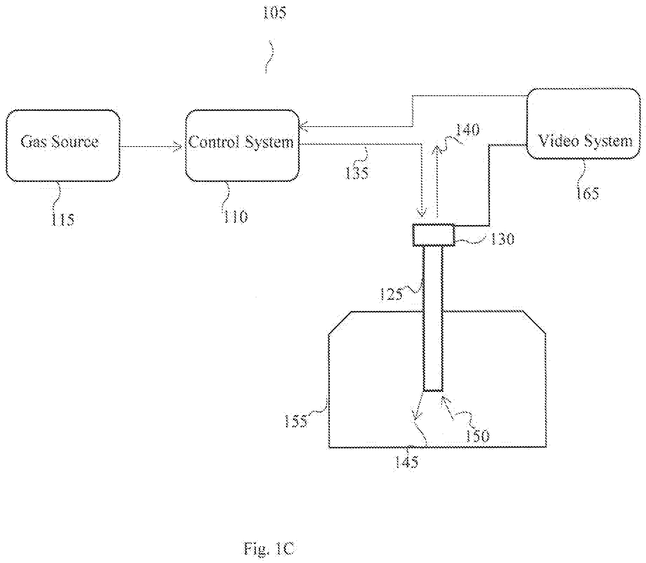

Fig. 1C discloses an MSID and a system for maintaining and controlling the gas pressure and flow within the body cavity, according to exemplary embodiments of the present invention. In the exemplary embodiment of figure 1C, the video system 165 communicates with both the MSID 125 and the control system 110. This way, the control system 110 may process and/or store information acquired or processed as a result of the video or images captured by the video system. Such information may be smoke detected in the body tissue, image clarity, distance between the MSID 125 and a predefined tissue or region of interest and the like. The control system 110 may use the information processed according to the video or images to control the gas flowing via the output mechanism 145 to the body tissue.

In some other cases, the video system 165 can process the video captured by the MSID 125 and detect smoke in the body cavity blocking the field of view. In such case, the video system 165 may instruct the control system 110 to evacuate or pump gas from the body cavity. Fig. ID discloses an MSID and a system for maintaining and controlling the gas pressure and flow within the body cavity, according to exemplary embodiments of the present invention. In this exemplary embodiment, the system comprises a sensor module 170 located in the MSID 125. The sensor module 170 may include a pressure sensor located on the external portion of the MSID 125 in the entrance and exit tubes. In some other cases, the pressure sensor on the body of the MSID 125, enabling the control system 110 to obtain the real conditions inside the body in real time.

In some cases flow and pressure sensors 170 may be located on gas path for accurate flow control.

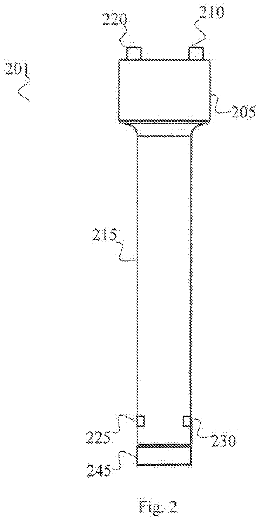

Fig. 2 shows an MSID comprising a Gas path apparatus, according to exemplary embodiments of the present invention. The Gas path apparatus 201 comprises a cylinder-shaped tube 215. The cylinder-shaped tube 215 is designed to be inserted into a body cavity in diverse medical procedures. The cylinder-shaped tube 215 comprises a gas exit port 225 designed to release gas into a body cavity. For example, in case there is a need to increase the gas pressure level within a body cavity in a certain medical procedure, the gas exit port 225 can be opened to allow pressurized gas to flow from the cylinder-shaped tube 215, through exit port 225, and into the body. The cylinder-shaped tube 215 also comprises a gas entry port 230 designed to receive pressurized gas from the body cavity and transfer it via the cylinder-shaped tube 215. For example, in case the gas pressure level within the body cavity is higher than the required gas pressure level in a certain medical procedure, the gas entry port 230 may be opened and receive pressurized gas into the cylinder-shaped tube 215.

The MSID comprising Gas path apparatus 201 also comprises a base 205 configured to contain a relatively large amount of pressurized gas. The base 205 comprises an entrance valve 220 designed to receive pressurized gas from the control system and allow the pressurized gas to flow further into the cylinder-shaped tube 215 in order to maintain the required gas pressure level within the cylinder-shaped tube 215. For example, in case a pressurized gas is delivered into a body cavity via gas exit port 225, the gas pressure level within the cylinder-shaped tube 215 is reduced. Thus, the entrance valve 220 can be opened to allow pressurized gas to flow into the cylinder-shaped tube 215. Once the gas pressure level has reached the required pressure level, the entrance valve 220 can be closed and block the gas from flowing into the cylinder-shaped tube 215. The required pressure level may be determined automatically or by the user who operates the medical procedure.

The base 205 also comprises a releasing valve 210 designed to release gas out of the cylinder-shaped tube 215 in order to maintain the required insufflation level as part of a certain medical procedure. For example, in case the pressurized gas is delivered from the body cavity to the cylinder-shaped tube 215, the gas pressure level within cylinder-shaped tube 215 increases. Thus, the releasing valve 210 is opened to release gas until the pressure level reaches the required pressure level.

The MSID also comprises a camera unit 245 configured to capture images of the vicinity of the MSID. The camera unit may also capture video. The images and/or video may be transmitted to another device for processing or display. The MSID may also comprise illumination module configured to illuminate the area captured by the camera unit 245.

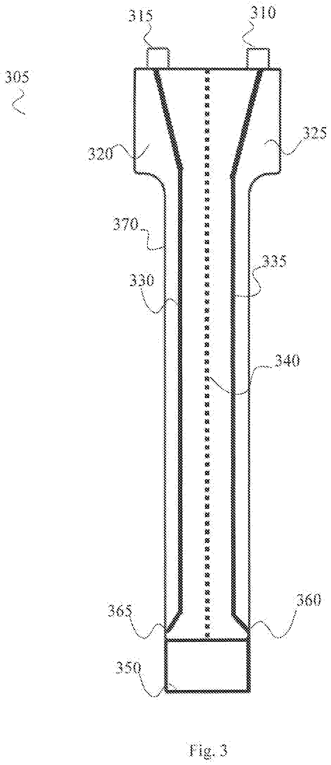

Fig. 3 shows a cross-section of an MSID and the gas delivery mechanism with gas entry and exit ports, not falling under the scope of the claimed subject-matter. Fig. 3 shows a cross-section of MSID 305 comprising a cylinder-shaped tube 370 designed to be inserted into a body cavity in diverse medical procedures as disclosed above. The MSID 305 also comprises an entrance gas path 320 through which gas flows into the body cavity. The entrance gas path 320 is defined between entrance port 315 and gas exit port 365 in which gas is dispensed into the body cavity. The entrance port 315 can be opened to allow pressurized gas to flow into entrance gas path 320 and thereby increase the gas pressure level therein. The gas contained in the entrance gas path 320 may be utilized to increase the gas pressure level in a body cavity. For example, in case pressurized gas was delivered into the body cavity, the gas pressure level within entrance gas path 320 may reach a pressure level that is lower than the required gas pressure level. Thus, the entrance port 315 may open and pressurized gas may flow into entrance gas path 320, until the gas pressure level meets the required pressure level. Then, in some cases, more pressurized gas can be used in order to increase the gas pressure level within the body cavity.

The MSID 305 also comprises a releasing port 310 utilized to control the gas pressure level by releasing gas out of the cylinder-shaped tube 370. The entrance port 315 and the releasing port 310 may include valves. MSID 305 also comprises a release gas path 325 located throughout the cylinder-shaped tube 370. The release gas path 325 is designed to contain the outlet of gas entry port 360 and to contain the gas which flows through entrance port 315. For example, releasing port 310 can be utilized to release a pressurized gas in order to reduce the gas pressure level to support the required insufflation level in the body cavity as part of a certain medical procedure. In such case, releasing port 310 can be opened to release a pressurized gas out of the cylinder-shaped tube 370 and thereby to reduce the gas pressure level within the release gas path 325.

In some cases, the cylinder-shaped tube 370 may comprise a first partition 330 configured to create an isolated and hermetic closed space for entrance gas path 320. The first partition 330 may also be designed to withstand pressures provided by the entrance gas path 320. Similarity, the cylinder-shaped tube 370 may also comprise a second partition 335 configured to create an isolated and hermetic closed space for gas path 325, and to withstand pressures provided by the gas path 325. In some cases, the gas pressure level within gas path 320 may be higher than the gas pressure level within the body cavity, and the gas pressure level within gas path 325 may be lower than the gas pressure level within the body cavity.

In some exemplary cases, the gas exit port 365 and gas entry port 360 are located at the lateral section of cylinder-shaped tube 370. Gas exit port 365 is located at the end of entrance gas path 320 and can be utilized to deliver pressurized gas from the entrance gas path 320 to the body cavity. In similar fashion, gas entry port 360 is located at the end of release gas path 325 and can be utilized to release pressurized gas from the body cavity to the gas path 325. In such embodiment of the present invention, the gas exit port 365 and the gas entry port 360 can be located above a module 350 and provide a relatively large number of optional camera types which can be placed at the camera module 350. Such camera types may be, a video camera, an x-ray camera, a digital image camera and the like.

Cylinder-shaped tube 370 may also comprise an electrical signal cable 340, designed to transmit the electrical signals from camera module 350 to an electrical transmitter in order to send it further to a display or to a computerized device. In some cases, such a transmitter may be designed to transmit video signals to communication networks, such as wireless LAN, Wi-Fi, Bluetooth, LAN, MAN, and the like. In some other cases, video signal transmitted via signal cable 340 may be connected to a digital processor in order to analyze the signals, and convert them to digital video streaming files. In some embodiments of the present invention, diverse type electrical cables may be added to the cylinder-shaped tube 370. Such cables may be power cables for the camera module, power cable to a lighting equipment installed at the vicinity of the camera module 350.

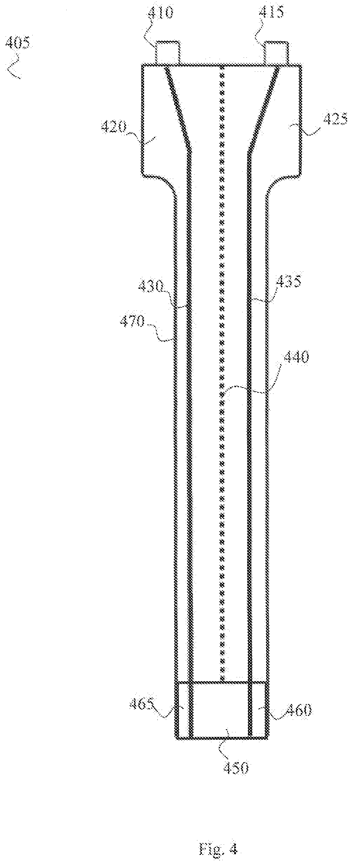

Fig. 4 shows a cross-section of an MSID and the gas delivery mechanism with gas entry and exit ports located at the front interface of the MSID, not falling under the scope of the claimed subject-matter. Fig. 4 shows a cross-section of MSID 405 comprising a cylinder-shaped tube 470 designed to be inserted into a body cavity. MSID 405 comprises an entrance gas path 420 designed to include the outlet of releasing valve 410 and a release gas path 425 designed to include the outlet of entrance valve 410 as disclosed above. In some cases, the releasing valve 410 or / and the entrance valve 415 may comprise gas pressure sensors and controllers in order to control the gas pressure level within the release gas path 425 and the entrance gas path 420. For example, gas pressure sensors may be utilized to measure the gas pressure within the entrance gas path 420 and in case the measured gas pressure is lower than the required pressure, the entrance valve 415 may be opened and allow the pressurized gas to flow into the gas path 420. In such cases, the pressurized gas in the entrance gas path 420 may be utilized to increase the gas pressure level within the body cavity. In a similar fashion, the releasing valve 410 may be utilized to release pressurized gas from gas path 425.

Fig. 4 demonstrates a possible embodiment of the present invention wherein the cylinder-shaped tube 470 comprises a gas entry port 460 and a gas exit port 465 located at the front surface of the cylinder-shaped tube 470, in the vicinity of the camera module 450. Gas exit port 465 can be utilized to deliver pressurized gas from the entrance gas path 420 to the body cavity. In a similar fashion, gas entry port 460 located near camera module 450 at the end of release gas path 425, is configured to release pressurized gas from the body cavity to the gas path 425. In such possible embodiment the gas exit port 465 and the gas entry port 460 can be located side by side to the camera module 450. The camera type of camera module 450 may be a video camera, an x-ray camera, a digital image camera and the like. Cylinder-shaped tube 470 also comprise an electrical signal cable 440, designed to transmit the electrical signals from camera module 450 to an electrical transmitter in order to send it further to a display, as disclosed above.

In some cases, the cylinder-shaped tube 470 may be provided in a shape of a half-cylinder tube which can be attached to a lateral interface of the MSID. In such cases the MSID may be also in a shape of a half-cylinder which creates a full cylinder shape by attaching to the half-cylinder tube of the replaceable gas path apparatus.

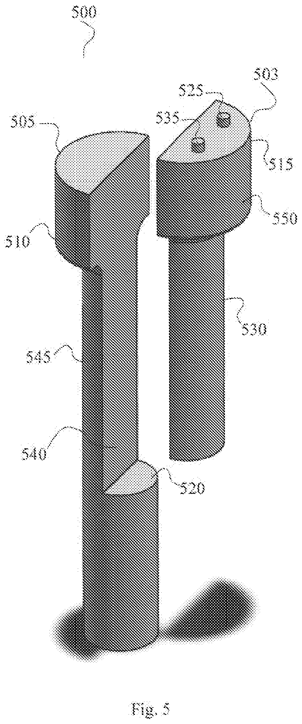

Fig. 5 shows a gas path apparatus in half cylinder-shaped tube designed to be attached to half-cylinder-shaped MSID, according to exemplary embodiments of the present invention. Fig. 5 shows a medical imaging device 505 provided in a half cylinder-shaped which can be attached to half cylinder-shaped Gas path apparatus 503 and thereby become a rounded MSID 500. MSID 500 is designed to be inserted into a body cavity and comprises gas path apparatus 503. The Gas path apparatus 503 comprises a half-rounded base 515 which comprises an entrance port 535 located at the top of a front surface of the half-rounded base 515. The entrance port 535 is designed to receive pressurized gas and allow it flow into the Gas path apparatus 503. The half-rounded base 515 also comprises a releasing port 525, also located at the top of the front surface of the half-rounded base 515 and designed to receive gas from the Gas path apparatus 503 and release it out. The half-rounded base 515 also comprises a lateral surface 550. In some embodiments of the present invention, entrance port 535 and releasing port 525 may be located at the lateral surface 550 of the half-rounded base 515. The Gas path apparatus 503 also comprises a half cylinder-shaped tube 530 extending downwardly from half-rounded base 515. In some cases, the half cylinder-shaped tube 530 may comprise a gas delivery mechanism and gas paths for delivering the pressurized gas during the medical procedures as elaborated above.

MSID 500 comprises a medical imaging device 505 can be attached to the replaceable surgical Gas path apparatus 503. Medical imaging device 505 comprises a half-round shaped base 510 which can be attached with the half-rounded base 515 of the Gas path apparatus 503 and form one rounded base. Medical imaging device 505 also comprises an elongated half-cylinder tube 545 designed to be attached with the half-cylinder-shaped tube 530 of the Gas path apparatus 503 and to jointly form a cylinder-shaped tube. In some cases, the attachment between medical imaging device 505 and the replaceable surgical Gas path apparatus 503 may be facilitated by a magnet, screws, bolts, welds, adhesive material, a mechanical clip on and the like. The medical imaging device 505 also comprises a flat surface 540 designed to be the contact interface with the replaceable surgical Gas path apparatus 503. For example, a user of MSID 500 can hold the replaceable surgical Gas path apparatus 503 and smoothly attach it to medical imaging device 505 in order to consolidate them into one unified MSID 500, Joining both parts into the unified MSID such to create a cylinder shape is important to allow a seal with the relevant surgical accessories, for example trocars. The medical imaging device 505 also comprises a supporting stair 520 positioned orthogonally to the flat surface 540. The supporting stair 520 may facilitate the attachment of the Gas path apparatus 503 by providing a basis which can support the Gas path apparatus 503 to be located in an accurate position.

In some embodiments of the present invention the medical imaging device 505 may comprise a part, or parts of the pressurized gas delivering mechanism of the MSID 500. For example, in some cases the medical imaging device 505 may comprise a gas path to deliver the pressurized gas to the human body. In such cases, the gas exit port or the gas entry port may be located at the surface of medical imaging device 505. In some cases, the flat surface 540 may comprise pipes or holes in order to receive the pressurized gas, delivered from replaceable surgical Gas path apparatus 503 and flow it into the body cavity. In such cases, the pipes or holes in flat surface 540 may also facilitate the gas releasing process out from the body.

In other possible embodiments of the present invention the supporting stair 520 may also comprise holes or valves designed to receive the pressurized gas from the Gas path apparatus 503, and to release the back the pressurized gas from the body cavity, to the Gas path apparatus 503. In some cases the Gas path apparatus 503 is a disposable part. In some cases Gas path apparatus 503 is a reusable part.

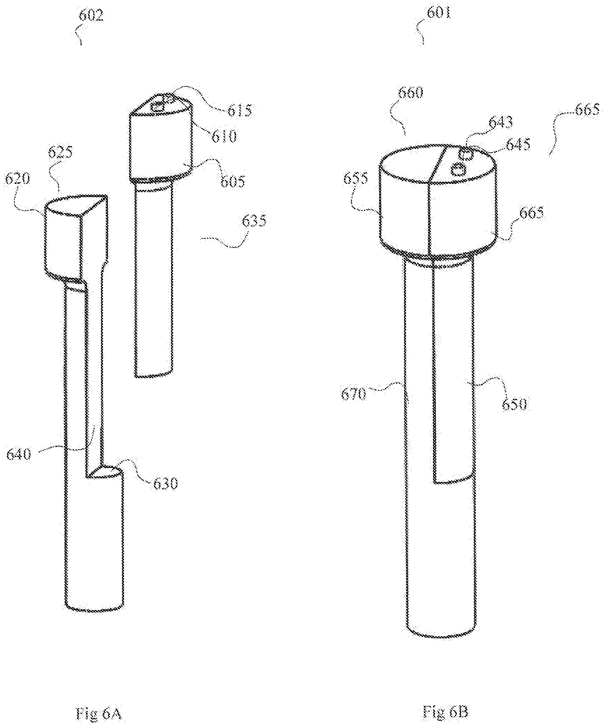

Figs. 6A and 6B show a replaceable surgical Gas path apparatus designed to be attached to a medical imaging device, according to exemplary embodiments of the present invention. Fig 6A shows a medical imaging device 625 which can be attached with a Gas path apparatus 635 and jointly become MSID 602. Medical imaging device 625 comprises a half-rounded base 620 which can be attached with half-rounded base 605 and jointly form one rounded base. Medical imaging device 625 also comprises a supporting stair 630 positioned orthogonally to a flat surface 640. The supporting stair 630 may facilitate the attachment act of the Gas path apparatus 635 by providing a basis which supports the replaceable surgical Gas path apparatus 635 to be located in an accurate position. The Gas path apparatus 635 also comprises a gas entrance port 610, and a gas releasing valve 615 located on the top surface of half-rounded base 605.

Fig. 6B shows a medical imaging device 660 attached with a Gas path apparatus 665 to provide a MSID 601. The Gas path apparatus 665 comprises a half-rounded base 665 which can form a round shaped base when attached to a half-rounded base 655, for example via a mechanical clip on or magnet. The Gas path apparatus 665 also comprises a half-cylinder-shaped tube 650 attached to an half-cylinder-shaped tube 670 and thereby form one round surface. The Gas path apparatus 665 also comprises a gas entrance port 643, and a gas releasing port 645 located at the top surface of half-rounded base 665.

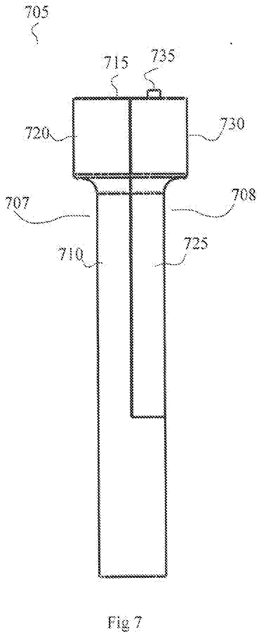

Fig. 7 shows a lateral view of a MSID, according to exemplary embodiments of the present invention. Fig. 7 shows a MSID 705 created by attaching a medical imaging device 707 with a Gas path apparatus 708. MSID 705 provides in a round, smooth, and seamless interface which can utilized in diverse medical procedures as elaborated above. MSID 705 comprises a base 715 formed by attaching a half-round base 730 to half-round base 720. MSID 705 also comprises an entrance port 735 located on the top of the base 715. In some cases, the valves utilized by the MSID 705 may be located at the top of the base 715. In some other cases, the ports may be located at the lateral surface of base 715. The base 715 may be of a round shape.

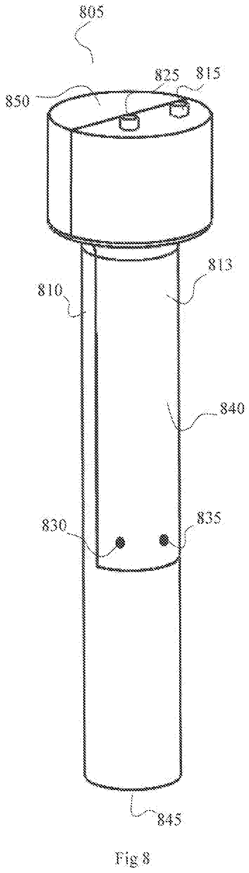

Fig. 8 demonstrates an angled view of a MSID having a Gas path apparatus and a medical imaging device, according to exemplary embodiments of the present invention. MSID 805 comprises a medical imaging device and 810 a Gas path apparatus 813. MSID 805 comprises a gas entrance port 825 and a gas releasing port 815 located on a top round surface 850. In some cases, gas entrance valve 825 and gas releasing valve 815 may comprise gas pressure sensors in order to control the gas pressure level.

A cylinder-shaped tube 840 of this exemplary embodiment comprises a gas exit port 830 and gas entry port 835 located at its lateral interface of the Gas path apparatus 813. In some cases, the exit port 830 may be utilized to deliver pressurized gas from the replaceable surgical Gas path apparatus 813 to the body cavity as part of a medical procedure. In similar fashion, gas entry port 835 can be utilized to release pressurized gas from the body cavity to the replaceable surgical Gas path apparatus 813 as part of a medical procedure. MSID 805 also comprises a front interface 845. In some cases, the gas exit port 830 and gas entry port 835 may be located at front interface 845. Thus, medical imaging device 810 may comprise additional gas delivering mechanism to deliver the pressurized gas from, or to the Gas path apparatus 813 and from, or to the body cavity. Such delivering mechanism may be pipes, gas paths, hollows to contain the pressurized gas, valves, and the like.

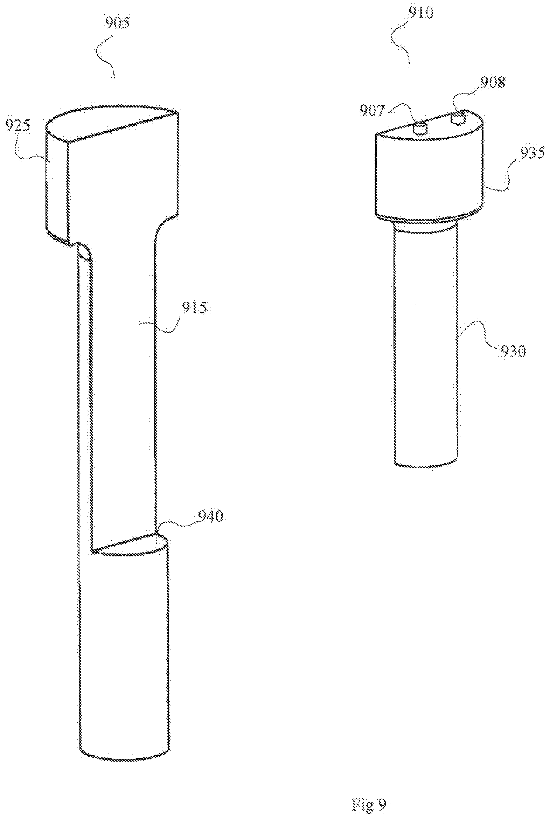

Fig. 9 shows an angled view of medical imaging device and a Gas path apparatus which can jointly become a MSID, according to exemplary embodiments of the present invention. Medical imaging device 905 may be designed to be attached with a Gas path apparatus 910 and comprises a flat surface 915 designed to be attached to the Gas path apparatus 910. In some cases, flat surface 915 may comprise a dedicated interface configured to accept the pressurized gas from the Gas path apparatus 910, in order to deliver the pressurized gas into a body cavity or a body hollow as part of a particular medical procedure.

Medical imaging device 905 also comprises a supporting stair 940 positioned orthogonally to the flat surface 915. The supporting stair 940 may facilitate the attachment of the Gas path apparatus 910 by providing a basis which supports the Gas path apparatus 910 in an accurate position. In some cases, a plurality of Gas path apparatus types may be configured and designed to be attached with the medical imaging device 905. In such cases, the medical imaging device 905 may be reusable in multiple medical procedures and the Gas path apparatus 910 may be replaced, in accordance with the requirements of the particular medical procedure. For example, in case a surgeon, or a user uses the medical imaging device 905 for a certain medical procedure, the user may be able to remove the Gas path apparatus 910 and attach a different type of Gas path apparatus, in order to perform the medical procedure. Such different type of replaceable surgical insufflators may be, replaceable surgical Gas path apparatuses with gas entry and exit ports located at different places at the replaceable surgical insufflators, replaceable surgical Gas path apparatus which support different types of pressurized gas, change in flow rate, different fittings to adopt to different insufflation systems and the like. The replaceable surgical insufflators 910 comprises a gas entrance port 907, and a gas releasing port 908 located on a top of a half round base 935. In some cases, gas entrance port 907 and gas releasing port 908 may switch their functionalities. In some cases, gas entrance port 907 and gas releasing port 908 may be constructed to be valves, thus, the gas releasing port 908 may become a gas entrance port and the gas entrance port 907 may become a gas releasing port. For example, in a specific medical procedure, the user of MSID may decide to change the gas flow direction by reconfiguring a system control component. Thus, the releasing port 908 may receive the pressurized gas from the system control and deliver it into the Gas path apparatus 910, and the entrance port 907 may release the pressurized gas out of the Gas path apparatus 910.

While the disclosure has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings without departing from the essential scope thereof. Therefore, it is intended that the disclosed subject matter not be limited to the particular embodiment disclosed as the best mode contemplated for carrying out this invention, but only by the claims that follow.

## Claims

1. A medical surgery imaging device (305, 500, 805) designed to be inserted into a patient's body, comprising:
a tube in the form of a cylinder-shaped tube, comprising
a medical imaging device (505), comprising
a half-round-shaped base (510) and
an elongated half cylinder tube (545), the elongated half cylinder tube (545) having a supporting stair (520) positioned orthogonally to a flat surface (540) of the elongated half cylinder tube (545),
a front interface (845),
a camera module (350, 450); and
a replaceable surgical gas path apparatus (503), comprising
a half-rounded base (515),
a half-cylinder-shaped tube (370, 530, 650,840),
an entrance gas path (320) located within the half-cylinder-shaped tube (370, 530) and defined between an entrance gas port (220,315, 535,610, 825, 907) and a gas exit port (225, 365, 830) in which gas is dispensed into a body cavity and
a release gas path (325) located within the half-cylinder-shaped tube (370, 530) and defined between a gas releasing port (210,310, 525, 615, 815, 908) and a gas entry port (230, 360, 835) in which gas is dispensed out of the body cavity,
**characterized in that** the half-rounded base (515) is configured to be attached to the half-round-shaped base (510) to form a top round surface (850, 715), and wherein the supporting stair (520) is configured to facilitate the half-cylinder-shaped tube (530) to be attached to elongated half cylinder tube (545) to jointly form the cylinder-shaped tube, wherein the attachment of the replaceable gas path apparatus (503) to the medical imaging device (505) forms the medical surgery imaging device (305, 500, 805) and wherein the gas path apparatus (503) is located inside the tube and designed to maintain a gas pressure level within said cavity or hollow in a body.

2. The medical surgery imaging device of claim 1, wherein the surgical gas path apparatus comprises the gas entry port which delivers pressurized gas into the body cavity.

3. The medical surgery imaging device of claim 1, further comprises a light source configured to be installed at a vicinity of the camera module (350) and designed to illuminate the vicinity of the medical surgery imaging device (305, 500, 805).

4. The medical surgery imaging device of claim 1, further configured to remove/detach the replaceable surgical gas path apparatus (503, 910) from the medical imaging device (505) and to attach a different type of replaceable gas path apparatus, such a different medical surgery imaging device (305, 500, 805) being designed to be inserted into the patient's body, wherein the gas entry port (230, 360, 835) and the gas exit port (225, 365, 830) are configured to be located at a different location along the half-cylinder-shaped tube (370, 530, 650,840).

5. A medical surgery system (105) controlling gas pressure within a body cavity, the system comprising:
the medical device (125,305,500,805) of one of the claims 1 to 4,
a gas interface (130) connected to a gas source (115) and configured to contain a gas entrance (135) and a gas releasing (140) to and from the medical surgery imaging device (125);
a control system (110) configured to receive gas from the gas source (115) and to deliver said gas to the gas interface (130);
a feedback module configured to receive an indication generated by a sensor located within the medical surgery imaging device (125), wherein the control system is configured to control the gas flow from the gas inlet into the body cavity according to the indication so as to maintain an insufflation level in the body cavity.

6. The medical surgery system of claim 5, wherein the sensor is a pressure sensor configured to detect pressure external to the medical imaging device and internal to the body cavity.

7. The medical surgery system of claim 5, wherein the sensor is a flow sensor configured to detect gas flowing in the surgical gas path apparatus.

8. The medical surgery system of claim 5, wherein the sensor is an image sensor.

## Patentansprüche

1. Eine medizinische Operations-Bildgebungsvorrichtung (305, 500, 805), die dazu ausgestaltet ist, in den Körper eines Patienten eingeführt zu werden, umfassend:
ein Rohr in Form eines zylinderförmigen Rohres, umfassend
ein medizinisches Bildgebungsgerät (505), umfassend
eine halbrunde Basis (510) und
ein längliches Halbzylinderrohr (545), wobei das längliche Halbzylinderrohr (545)
eine Stützstufe (520) aufweist, die orthogonal zu einer flachen Oberfläche (540) des länglichen Halbzylinderrohrs (545) angeordnet ist,
eine vordere Schnittstelle (845),
ein Kameramodul (350, 450); und
eine austauschbare Operations-Gaswegvorrichtung (503), umfassend
eine halbrunde Basis (515),
ein halbzylinderförmiges Rohr (370, 530, 650, 840),
einen Gaseingangsweg (320), der sich innerhalb des halbzylinderförmigen Rohrs (370, 530) befindet und zwischen einem Gaseingangsanschluss (220, 315, 535, 610, 825, 907) und einem Gasausgangsanschluss (225, 365, 830) definiert ist, in dem Gas in einen Körperhohlraum abgegeben wird und
einen Gasabgabeweg (325), der sich innerhalb der halbzylinderförmigen Röhre (370, 530) befindet und zwischen einem Gasaustrittanschluss (210, 310, 525, 615, 815, 908) und einem Gaseintrittsanschluss (230, 360, 835) definiert ist, in dem Gas aus dem Körperhohlraum abgegeben wird,
**dadurch gekennzeichnet, dass** die halbrunde Basis (515) dazu eingerichtet ist, an der halbrunden Basis (510) angebracht zu werden, um eine obere runde Fläche (850, 715) zu bilden, und wobei die Stützstufe (520) dazu eingerichtet ist, die Anbringung des halbzylinderförmigen Rohrs (530) an dem länglichen Halbzylinderrohr (545) zu erleichtern, um gemeinsam das zylinderförmige Rohr auszubilden, wobei die Befestigung der austauschbaren Gaswegvorrichtung (503) an dem medizinischen Bildgebungsgerät (505) die medizinische Operations-Bildgebungsvorrichtung (305, 500, 805) ausbildet und wobei die Gaswegvorrichtung (503) im Inneren des Rohrs angeordnet und so ausgestaltet ist, dass sie ein Gasdruckniveau innerhalb des Hohlraums oder der Höhlung in einem Körper aufrechterhält.

2. Die medizinische Operations-Bildgebungsvorrichtung nach Anspruch 1, wobei die Operations-Gaswegvorrichtung den Gaseintrittsanschluss umfasst, der unter Druck stehendes Gas in den Körperhohlraum liefert.

3. Die medizinische Operations-Bildgebungsvorrichtung nach Anspruch 1, ferner umfassend eine Lichtquelle, die dazu eingerichtet ist, in der Nähe des Kameramoduls (350) angebracht zu werden und die Umgebung der medizinischen Operations-Bildgebungsvorrichtung (305, 500, 805) zu beleuchten.

4. Die medizinische Operations-Bildgebungsvorrichtung nach Anspruch 1, das weiterhin dazu eingerichtet ist, die austauschbare Operations-Gaswegvorrichtung (503, 910) von dem medizinischen Bildgebungsgerät (505) zu entfernen/abzunehmen und einen anderen Typ einer austauschbaren Gaswegvorrichtung anzubringen, solch eine andere medizinisch Operations-Bildgebungsvorrichtung (305, 500, 805), die dazu ausgebildet ist, in den Körper des Patienten eingeführt zu werden, wobei der Gaseintrittsanschluss (230, 360, 835) und der Gasausgangsanschluss (225, 365, 830) so eingerichtet sind, dass sie sich an einer anderen Stelle entlang des halbzylinderförmigen Rohrs (370, 530, 650, 840) befinden.

5. Ein medizinisches Operationssystem (105) zur Steuerung des Gasdrucks in einem Körperhohlraum, wobei das System umfasst:
die medizinische Vorrichtung (125, 305, 500, 805) nach einem der Ansprüche 1 bis 4,
eine Gasschnittstelle (130), die mit einer Gasquelle (115) verbunden und dazu eingerichtet ist, einen Gaseintritt (135) und einen Gasaustritt (140) zu und von der medizinischen Operations-Bildgebungsvorrichtung;
ein Steuersystem (110), das dazu eingerichtet ist, Gas von der Gasquelle (115) zu empfangen und das Gas an die Gasschnittstelle (130) zu liefern;
ein Rückkopplungsmodul, das dazu eingerichtet ist, eine Anzeige zu empfangen, die von einem Sensor erzeugt wird, der sich in der medizinischen Operations-Bildgebungsvorrichtung (125) befindet, wobei das Steuersystem dazu eingerichtet ist, den Gasfluss von dem Gaseinlass in den Körperhohlraum entsprechend der Anzeige zu steuern, um ein Insufflationsniveau in dem Körperhohlraum aufrechtzuerhalten.

6. Das medizinische Operationssystem nach Anspruch 5, wobei der Sensor ein Drucksensor ist, der dazu eingerichtet ist, den Druck außerhalb des medizinischen Bildgebungsgeräts und innerhalb des Körperhohlraums zu erfassen.

7. Das medizinische Operationssystem nach Anspruch 5, wobei der Sensor ein Durchflusssensor ist, der dazu eingerichtet ist, in der Operations-Gaswegvorrichtung strömendes Gas zu erfassen.

8. Das medizinische Operationssystem nach Anspruch 5, wobei der Sensor ein Bildsensor ist.

## Revendications

1. Dispositif médical d'imagerie chirurgicale (305, 500, 805) conçu pour être inséré dans le corps d'un patient, comprenant :
un tube sous la forme d'un tube de forme cylindrique, comprenant
un dispositif médical d'imagerie (505), comprenant
une base de forme semi-circulaire (510) et
un tube semi-cylindrique allongé (545), le tube semi-cylindrique allongé (545) ayant une marche de support (520) positionnée de manière orthogonale à une surface plate (540) du tube semi-cylindrique allongé (545),
une interface avant (845),
un module de caméra (350, 450) ; et
un appareil à trajet de gaz chirurgical remplaçable (503), comprenant une base semi-circulaire (515),
un tube de forme semi-cylindrique (370, 530, 650, 840),
un trajet de gaz d'entrée (320) situé au sein du tube de forme semi-cylindrique (370, 530) et défini entre un port de gaz d'entrée (220, 315, 535, 610, 825, 907) et un port de sortie de gaz (225, 365, 830) dans lequel du gaz est distribué dans une cavité corporelle et
un trajet de gaz de libération (325) situé au sein du tube de forme semi-cylindrique (370, 530) et défini entre un port de libération de gaz (210, 310, 525, 615, 815, 908) et un port d'entrée de gaz (230, 360, 835) dans lequel du gaz est distribué hors de la cavité corporelle,
**caractérisé en ce que** la base semi-circulaire (515) est configurée pour être reliée à la base de forme semi-circulaire (510) pour former une surface ronde supérieure (850, 715), et dans lequel la marche de support (520) est configurée pour faciliter au tube de forme semi-cylindrique (530) d'être relié au tube semi-cylindrique allongé (545) pour former conjointement le tube de forme cylindrique, dans lequel la liaison de l'appareil à trajet de gaz remplaçable (503) au dispositif médical d'imagerie (505) forme le dispositif médical d'imagerie chirurgicale (305, 500, 805) et dans lequel l'appareil à trajet de gaz (503) est situé à l'intérieur du tube et conçu pour maintenir un niveau de pression gazeuse au sein de ladite cavité ou dudit creux dans un corps.

2. Dispositif médical d'imagerie chirurgicale selon la revendication 1, dans lequel l'appareil à trajet de gaz chirurgical comprend le port d'entrée de gaz qui fournit du gaz pressurisé dans la cavité corporelle.

3. Dispositif médical d'imagerie chirurgicale selon la revendication 1, comprenant en outre une source lumineuse configurée pour être installée à proximité du module de caméra (350) et conçue pour éclairer la proximité du dispositif médical d'imagerie chirurgicale (305, 500, 805).

4. Dispositif médical d'imagerie chirurgicale selon la revendication 1, configuré en outre pour retirer/déconnecter l'appareil à trajet de gaz chirurgical remplaçable (503, 910) du dispositif médical d'imagerie (505) et pour relier un type différent d'appareil à trajet de gaz remplaçable, un tel dispositif médical d'imagerie chirurgicale différent (305, 500, 805) étant conçu pour être inséré dans le corps du patient, dans lequel le port d'entrée de gaz (230, 360, 835) et le port de sortie de gaz (225, 365, 830) sont configurés pour être situés à un emplacement différent le long du tube de forme semi-cylindrique (370, 530, 650, 840).

5. Système médical chirurgical (105) commandant la pression gazeuse au sein d'une cavité corporelle, le système comprenant :
le dispositif médical (125, 305, 500, 805) selon une des revendications 1 à 4,
une interface gazeuse (130) connectée à une source gazeuse (115) et configurée pour contenir une entrée de gaz (135) et une libération de gaz (140) vers et depuis le dispositif médical d'imagerie chirurgicale (125) ;
un système de commande (110) configuré pour recevoir du gaz de la source gazeuse (115) et pour fournir ledit gaz à l'interface gazeuse (130) ;
un module de rétroaction configuré pour recevoir une indication générée par un capteur situé au sein du dispositif médical d'imagerie chirurgicale (125), dans lequel le système de commande est configuré pour commander l'écoulement gazeux de l'entrée de gaz dans la cavité corporelle en fonction de l'indication de manière à maintenir un niveau d'insufflation dans la cavité corporelle.

6. Système médical chirurgical selon la revendication 5, dans lequel le capteur est un capteur de pression configuré pour détecter une pression externe au dispositif médical d'imagerie et interne à la cavité corporelle.

7. Système médical chirurgical selon la revendication 5, dans lequel le capteur est un débitmètre configuré pour détecter du gaz s'écoulant dans l'appareil à trajet de gaz chirurgical.

8. Système médical chirurgical selon la revendication 5, dans lequel le capteur est un capteur d'image.
